# EUROPÄISCHE PATENTSCHRIFT

(11) **EP 0 939 607 B1**
(45) Veröffentlichungstag und Bekanntmachung des Hinweises auf die Patenterteilung: **30.05.2001**
(21) Anmeldenummer: 97953758.6
(22) Anmeldetag: 09.12.1997
(51) Int. Cl.: A61F 2/42

(54) **KÜNSTLICHES FINGERGELENK**
ARTIFICIAL FINGER JOINT
ARTICULATION ARTIFICIELLE DU DOIGT

(30) Priorität: 09.12.1996 DE 19650816
(43) Veröffentlichungstag der Anmeldung: 08.09.1999
(73) Patentinhaber: ESKA Implants GmbH & Co., 23556 Lübeck (DE)
(72) Erfinder: GRUNDEI, Hans, D-23558 Lübeck (DE); BLESSE, Jörg, D-23564 Lübeck (DE)
(74) Vertreter: Fuchs Mehler Weiss & Fritzsche
(86) Internationale Anmeldenummer: EP9706853
(87) Internationale Veröffentlichungsnummer: WO9825551

(56) Entgegenhaltungen:
- EP-A- 0 518 278
- WO-A-89/03663
- DE-A- 1 960 087
- DE-A- 3 741 488
- DE-C- 19 512 854
- FR-A- 2 158 657
- FR-A- 2 736 536
- GB-A- 1 582 450
- US-A- 3 990 116
- US-A- 5 534 033

## Beschreibung

Die vorliegende Erfindung betrifft ein Fingergelenk zur Überbrückung eines natürlichen, resezierten Fingergelenks mit zwei implantierbaren Stielteilen, die beide mittels eines biegbaren Verbindungselementes verbindbar sind.

Künstliche Fingergelenke sind beispielsweise bekannt aus der DE-A-19 60 087, der WO-A-89/03663 und der GB-A-15 82 450. Diese Fingergelenke werden insbesondere zwischen dem Phalanx mediae und dem Phalanx prox. implantiert. Das durch ein Fingergelenk gemäß dem Stand der Technik zu ersetzende Fingermittelgelenk ähnelt stark dem Aufbau und in der Funktion dem natürlichen Kniegelenk, bei dem eine Gleit- und Rollbewegung der Gelenkteile zueinander ausgeführt wird. Das Fingermittelgelenk ist so aufgebaut, daß es gebeugt und gestreckt werden kann durch Muskelkraft, die über Streck- und Beugesehnen auf die Fingersegmente übertragen werden. Kommt es hier zu einem Strecksehnenabriß, ist eine Operation durch Wiederanbindung der Strecksehne an dem betroffenen Fingersegment möglich.

Wird bei einem Patienten ein Strecksehnenanriß am Fingerendgelenk diagnostiziert, so äußert sich dies in der für den Patienten unangenehmen Weise dadurch, daß er zwar das Endgelenk des betroffenen Fingers beugen kann, allerdings keine Möglichkeit besteht, den Finger wieder voll zu strecken. Dies eben aufgrund der fehlenden Zugkräfte der Strecksehne. Eine Wiederanbindung der abgerissenen Strecksehne ist im Bereich des Fingerendgelenks aber praktisch unmöglich.

Diese bekannten Fingergelenke versuchen dieses Problem durch Verwendung eines elastischen Silikongummis als Verbindungselement zwischen den zu implantierenden Stielteilen zu lösen.

Die Materialdicke des Silikongummis kann aber einer Beugebewegung des Gelenkes entgegenstehen, aufgrund der erforderlichen Materialverdrängung. Dies beeinträchtigt die Bewegbarkeit des Gelenkes.

Aufgabe der vorliegenden Erfindung ist es daher, ein Fingergelenk für den Ersatz eines Fingerendgelenkes der eingangs genannten Art so weiterzubilden, daß mit seiner Hilfe ein Finger bei Strecksehnenabriß leicht wieder in gestreckte Lage gebracht werden kann bei ansonsten gegebener guter Beweglichkeit.

Gelöst wird die Aufgabe durch das Fingergelenk mit den Merkmalen des Anspruchs 1. Eine vorteilhafte Weiterbildung ergibt sich aus dem Anspruch 2.

Dementsprechend ist vorgesehen, daß das Verbindungselement aus einer Vielzahl von Silikonstäben besteht, welche in entsprechende Bohrungen in den Stielteilen setzbar sind. Dies Silikon ist in seiner Shore-Härte so einzustellen, daß es die Rückstellkräfte auf die Beugung des Gelenkes hin erzeugen kann.

Das Verbindungselement entwickelt auf eine Beugung des Gelenkes hin die Rückstellkräfte, welche das Gelenk in die gestreckte Lage drängen.

Die Ruhelage oder die entspannte Lage des erfindungsgemäßen Fingergelenks ist also die gestreckte Lage. Aufgrund dieser Eigenschaft wird die Funktion der abgerissenen Strecksehne nachempfunden.

Zur Implantation wird die Gelenkkapsel des betroffenen Endgelenkes reseziert derart, daß die Kondylen und Laufflächen des natürlichen Gelenks entfernt werden. Dann werden die Knochen Phalanx mediae und Phalanx distalis zur Aufnahme jeweils eines der beiden Stielteile präpariert, d.h. ausgefräst. In die Aushöhlung wird jeweils ein Stielteil gesetzt, entweder unter Anwendung eines Knochenzements, wie Polymethylmetacrylat oder zementlos, wobei dann die Oberflächen der Stielteile speziell ausgebildet sein müssen, wie weiter unten näher erläutert wird.

Die Stielteile bestehen im übrigen vorzugsweise aus körperverträglichem Metall.

Für eine zementlose Fixation der Stielteile in den Knochen ist es besonders vorteilhaft, wenn die Oberfläche der Stielteile zumindest bereichsweise mit einer dreidimensionalen, offenmaschigen Raumnetzstruktur belegt ist, welche auch als interkonnektierend bezeichnet wird. In diese Oberflächenstruktur kann Knochenmaterial hineinwachsen und die Stege oder Maschenwendeln umwachsen, so daß dementsprechend auch ein Substratfluß in die räumliche Tiefe der Oberfläche hinein stattfinden kann. Diese Strukturoberfläche dient zum dauerhaften Einwachsen von Knochenmaterial in die Implantate, woraus eine hervorragende Langzeitstabilität resultiert.

Die Erfindung wird nun anhand von Ausführungsbeispielen gemäß der Zeichnungsfiguren näher erläutert. Hierbei zeigt:
- Fig. 1: schematisch die Implantationslage der Stielteile in einem Finger und
- Fig. 2: eine perspektivische Ansicht des Gelenkes.

Nachfolgend sind entsprechende Teile beider Ausführungsbeispiele des Fingergelenks mit denselben Bezugszeichen versehen.

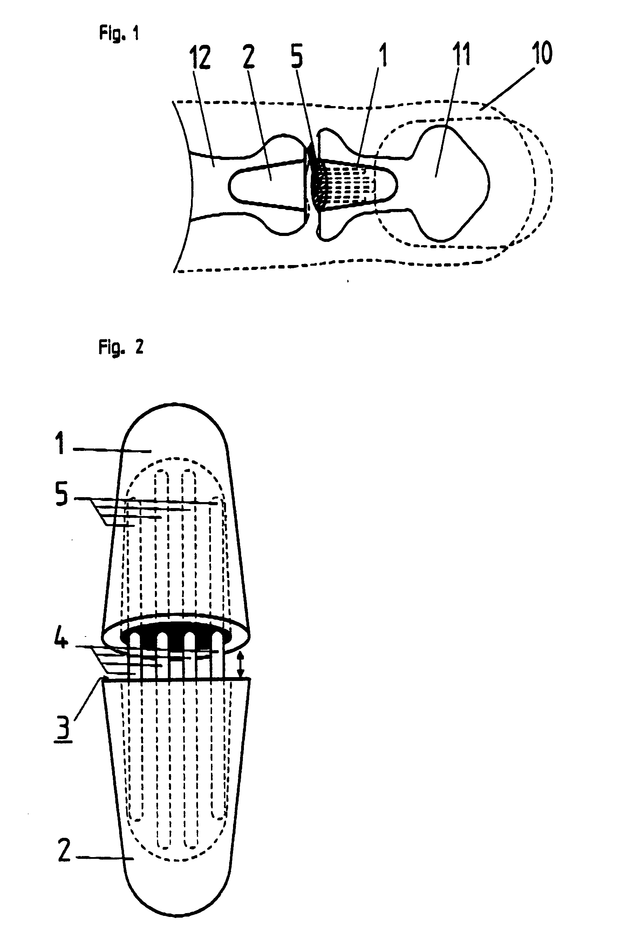

Fig. 1 veranschaulicht die Implantationsposition des Fingergelenks in situ. Der von einem Strecksehnenabriß in Mitleidenschaft gezogenen Finger 10 wird operativ geöffnet und im Phalanx mediae 12 und im Phalanx distalis 11 jeweils eine Ausfräsung vorgenommen. In die Ausfräsungen werden zwei Stielteile 1 und 2 des Fingergelenkes gesetzt. Diese weisen an ihrer Basis Bohrungen 5 auf für die Aufnahme von biegsamen Stäben 4 auf (Fig. 2), welche die benötigten Rückstellkräfte erzeugen, wenn das äußere Fingerglied angewinkelt wird. Einzelheiten lassen sich aus der Fig. 2 ersehen. Vorliegend sind vier Stäbe 4 vorgesehen, welche zusammen das Verbindungselement 3 bilden. Die Rückstellkräfte des Fingergelenks lassen sich individuell einstellen durch Auswahl des Materials für die Stäbe 4 sowie durch Wahl von deren Dicke. Die Stäbe werden in das Innere der Stielteile 1 und 2 in die Bohrungen 5 gesetzt. Bohrungsdurchmesser und Stabdurchmesser sind so aufeinander abgestimmt, daß ein dauerhafter Halt gegeben ist.

## Patentansprüche

1. Fingergelenk zur Überbrückung eines natürlichen, resezierten Fingergelenks mit zwei implantierbaren Stielteilen (1, 2), die beide mittels eines biegbaren Verbindungselementes (3) verbindbar sind, wobei ein Stielteil (1) im Phalanx mediae und das andere Stielteil (2) im Phalanx distalis verankerbar ist, und das Verbindungselement (3) auf eine Beugung des Gelenkes hin Rückstellkräfte entwickelt, welche das Gelenk in die gestreckte Lage drängen,
dadurch gekennzeichnet,
daß das Verbindungselement (3) aus einer Mehrzahl von Silikonstäben (4) besteht, die in entsprechende Bohrungen (5) in den Stielteilen (1,2) setzbar sind.

2. Fingergelenk nach Anspruch 1, bei dem die Oberfläche der Stielteile (1, 2) zumindest bereichsweise mit einer dreidimensionalen, offenmaschigen Raumnetzstruktur (7) belegt ist.

## Claims

1. Finger joint for bridging a natural, resected finger joint having two implantable stem parts (1, 2), which can both be connected by means of a bendable connecting element (3), wherein one stem part (1) can be anchored in the phalanx mediae and the other stem part (2) in the phalanx distalis, and the connecting element (3) develops restoring forces on bending of the joint, which press the joint into the extended position,
characterised in that the connecting element (3) comprises a plurality of silicone rods (4) which can be placed in corresponding bores (5) in the stem parts (1, 2).

2. Finger joint according to claim 1, in which the surface of the stem parts (1, 2) is covered at least partly with a three-dimensional, open-mesh spatial network structure (7).

## Revendications

1. Articulation de doigt pour le remplacement d'une articulation de doigt naturelle réséquée, avec deux parties de tiges implantables (1, 2) pouvant être reliées l'une et l'autre au moyen d'un élément de liaison flexible (3), dans laquelle une partie de tige (1) peut être ancrée dans la deuxième phalange et l'autre partie de tige (2) dans la troisième phalange et, suite à une flexion de l'articulation, l'élément de liaison (3) exerce des forces de rappel qui forcent l'articulation en position d'extension, caractérisée en ce que l'élément de liaison (3) se compose d'une pluralité de joncs en silicone (4) qui peuvent être introduits dans des perçages (5) correspondants dans les parties de tiges (1, 2).

2. Articulation de doigt selon la revendication 1, dans laquelle la surface des parties de tiges (1, 2) est revêtue au moins par endroits avec une structure déployée à mailles ouvertes en trois dimensions.
